**Europäisches Patentamt**

**European Patent Office**

**Office européen des brevets**

(11) Veröffentlichungsnummer: **0 402 757**
**A2**

(12) **EUROPÄISCHE PATENTANMELDUNG**

(21) Anmeldenummer: **90110734.2**

(22) Anmeldetag: **06.06.90**

(51) Int. Cl.5: **G01N 33/543, G01N 33/58, G01N 33/569, G01N 33/576**

(30) Priorität: **16.06.89 DE 3919810**

(43) Veröffentlichungstag der Anmeldung:
**19.12.90 Patentblatt 90/51**

(84) Benannte Vertragsstaaten:
**AT BE CH DE DK ES FR GB GR IT LI NL SE**

(71) Anmelder: **Biochrom Beteiligungs GmbH &**
**Co. Produktionsgesellschaft**
**Leonorenstrasse 2-6**
**D-1000 Berlin 46(DE)**

Anmelder: **VEB Sächsisches Serumwerk**
**Dresden**
**Herbert-Bochow-Strasse 40**
**DDR-8010 Dresden(DD)**

(72) Erfinder: **Von Baehr, Rüdiger, Prof. Dr.**
**Märkisches Ufer 22**
**DDR-1020 Berlin(DE)**
Erfinder: **Döpel, Holger, Dr.**
**Linienstrasse 88**
**DDR-1054 Berlin(DE)**
Erfinder: **Frenzel, Bernd, Dr.**
**Lorenzstrasse 65**
**D-1000 Berlin 45(DE)**
Erfinder: **Kluge, Manfred**
**Blochmannstrasse 1**
**DDR-8010 Dresden(DE)**
Erfinder: **Nagel, Elsa, Ing.-Chem.**
**Sebastianstrasse 35**
**DDR-1020 Berlin(DE)**
Erfinder: **Porstmann, Bärbel, Prof., Dr.**
**Schneeglöckchenstrasse 29**
**DDR-1055 Berlin(DE)**
Erfinder: **Porstmann, Thomas, Prof., Dr.**
**Märkisches Ufer 22**
**DDR-1020 Berlin(DE)**

(74) Vertreter: **Henkel, Feiler, Hänzel & Partner**
**Möhlstrasse 37**
**D-8000 München 80(DE)**

(54) **Festphasenimmunoassay und Testbesteck zur Bestimmung von Antigenen und Antikörpern.**

(57) Die Erfindung betrifft ein Verfahren zum kombinierten Nachweis einer Infektion mit dem Hepatitis B Virus und/oder einer Infektion mit den beiden Varianten des humanen Immuninsuffizienz-Virus HIV-1 und/oder HIV-2. Zur Unterbrechung von Infektionsketten und Verhinderung der Übertragung dieser Viren mit Blut- und Blutprodukten findet das Verfahren Anwendung in der Humanmedizin und hier vorrangig im Blutspendewesen und der klinisch-chemischen sowie virologischen Diagnostik.

EP 0 402 757 A2

## Festphasenimmunoassay und Testbesteck zur Bestimmung von Antigenen und Antikörpern

Anwendung der Erfindung

Die Erfindung betrifft ein Verfahren zum kombinierten Nachweis einer Infektion mit dem Hepatitis B Virus und/oder einer Infektion mit den beiden Varianten des humanen Immuninsuffizienz-Virus HIV-1 und/oder HIV-2. Zur Unterbrechung von Infektionsketten und Verhinderung der Übertragung dieser Viren mit Blut- und Blutprodukten findet das Verfahren Anwendung in der Humanmedizin und hier vorrangig im Blutspendewesen und der klinisch-chemischen sowie virologischen Diagnostik.

Charakteristik des bekannten Standes der Technik

Der Nachweis der Infektion mit diesen Viren wird ausschließlich durch serologische Untersuchungen geführt. Als Methode dominiert dabei der Enzyme linked Immunosorbent Assay (ELISA). Die Diagnostik der Hepatitis B Virusinfektion basiert dabei auf dem Nachweis von im Überschuß produziertem Hüllantigen (Hepatitis B Oberflächenantigen, HBsAg), welches in die Blutbahn sekretiert wird. Der Nachweis einer Infektion mit den Retroviren HIV-1 und/oder HIV-2 als Verursacher der erworbenen Immunschwäche AIDS kann gegenwärtig nur indirekt über die Erfassung induzierter virusspezifischer Antikörper geführt werden.

Bis jetzt erfolgt die Testung auf Infektion mit diesen Erregern in getrennten Immunoassays, die unabhängig voneinander durchgeführt werden, wobei jeder dieser Teste einer individuellen Bearbeitung und Auswertung bedarf.

Für den Nachweis des HBsAg werden als Screeningsysteme fast ausnahmslos Zwei-Seiten Bindungsassays (Schuurs, A.H.W.M. und B.K. van Weemen: Clin. Chim. Acta 81 (1977), 1-40) unter Einsatz sowohl polyklonaler als auch monoklonaler Antikörper entweder als sukzessive Zwei-Schritt Techniken oder als simultane Ein-Schritt Techniken eingesetzt. Aufgrund der geforderten Nachweisempfindlichkeit von 1 $\mu$g HBsAg/1 werden die zu untersuchenden Seren entweder unverdünnt oder im Falle der simultanen Ein-Schritt Technik durch Konjugatzusatz maximal 1 : 2 verdünnt eingesetzt.

Antikörper gegen HIV-1 und HIV-2 können prinzipiell nach 4 verschiedenen Verfahren nachgewiesen werden: Der Antiglobulin-oder Sandwichtechnik, der Kompetitionstechnik, der Capturetechnik und der Brückenantikörpertechnik (R. Tedder, in: Acquired Immuno Deficiency Syndrome. Hrsg. J.C. Gluckman und E. Vilmer. Elsevier; Amsterdam, New York, Oxford, Paris. 1986, S. 173-181). Dabei hat sich bis auf wenige Ausnahmen die Antiglobulintechnik für die Screeninguntersuchungen durchgesetzt. Um falsch positive Resultate durch unspezifische IgG Adsorptionen an der festen Phase zu minimieren oder durch Reaktionen mit Proteinen lymphozytärer Herkunft in den Testen der ersten Generation auf der Basis von Viruslysat zu verhindern, erfordert diese Technik im allgemeinen Serumverdünnungen von mindestens 1 : 21 bis 1 : 100.

Aufgrund der zu erwartenden Entdeckungen weiterer humanphatogener Erreger ist zukünftig mit einer Erweiterung der Parameterzahl zu rechnen, auf die eine Blutkonserve getestet werden muß, bevor sie zur Transfusion oder weiteren Verarbeitung freigegeben werden kann.

Deshalb gibt es erste Bestrebungen, Screeningteste zu vereinen, um mehrere Parameter mit einem Test zu erfassen. Eine Differenzierung war jedoch bisher nicht möglich (L. Nimms, B. Braunn, K. Mayer, S. Earle und L. Valdivia: III Internationale Konferenz über AIDS, Washington, 1987. Poster MP: 244; Assay for detecting hepatitis antigens and aids antibodies, Europa-Patent 28 62 64). Damit wird bei einem positiven Befund die Blutkonserve für den weiteren Gebrauch zwar gesperrt, aber gleichzeitig werden Wiederholungsuntersuchungen in getrennten Testen zur Differentialdiagnostik notwendig. Außerdem setzen solche Teste zur parallelen Bestimmung mehrerer Faktoren auf der Basis eines Markerenzyms einen einheitlichen Extinktionsgrenzwert, der mitunter ganz differente Substanzkonzentrationen repräsentieren muß, voraus, was nur durch Kompromißlösungen entweder auf Kosten der Spezifität oder der Sensitivität des einen oder des anderen Parameters zu realisieren ist. Darüberhinaus wird die Kombinationsmöglichkeit der Assayvarianten, in denen die unterschiedlichen Substanzen unter Verwendung verschiedener, mit nur einem Markerenzym markierten Reaktanten nachgewiesen werden, erheblich dadurch eingeschränkt, daß das Farbsignal in seiner Intensität sich für die nachzuweisenden Substanzen unbedingt gleichsinnig verändern muß (entweder für beide direkt oder indirekt proportional zur Substanzkonzentration).

Ziel der Erfindung

Der Erfindung lag die Aufgabe zugrunde, Antigen bakterieller oder viraler Infektionserreger sowie spezifischer Antikörper gegen mehrere Infektionserreger in ein und demselben Testansatz differenziert nachzuweisen, um damit einerseits die Zahl der Ansätze gegenüber der Anwendung bisher verfügbarer Testsysteme zu reduzieren und andererseits die erforderlichen Nachuntersuchungen zur Differentialdiagnostik in getrennten Testsystemen zu umgehen.

Die prinzipielle Lösung der Aufgabe besteht darin, einen Festphasen-Enzymimmunoassay mit simultaner Ein-Schritt- oder sukzessiver Zwei-Schritt-Inkubationstechnik zum Antigennachweis und/oder Nachweis spezifischer Antikörper zu kombinieren, was die Koimmobilisierung von Antikörpern und/oder Virusproteinen an der festen Phase und den Einsatz von mit verschiedenen Enzymen markierten Reaktanten voraussetzt.

Dabei wird für den Antigennachweis ein Markerenzym-1 zur Markierung der Antikörper gewählt, dessen optimale Substratreaktionsbedingungen sowie das gebildete Produkt weder die Proteine denaturieren noch die über die adsorbierten Peptide gebundenen Immunkomplexe an der festen Phase für den zweiten Teil des Testes, den Nachweis spezifischer antiviraler Antikörper, zerstören. Nach Ablauf der gemeinsamen Inkubation von zu untersuchender Probe und Enzym-1 markierten Antikörpern im Falle der simultanen Inkubationstechnik kann nach Abtrennung der ungebundenen Reaktanten nach bekanntem Verfahren durch Substratreaktion bereits entschieden werden, ob das zu bestimmende Antigen vorhanden ist oder nicht. Nach Entfernung der Substratlösung werden im zweiten Inkubationsschritt mit Markerenzym-2 markierten anti-human IgG- Antikörpern die Bindung spezifischer antibakterieller oder antiviraler humaner Antikörper über eine weitere Substratreaktion nachgewiesen.

Durch den Einsatz von zwei Enzymen mit unterschiedlichen Substraten ist die Differenzierungsmöglichkeit zwischen nachzuweisendem Antigen und zu erfassenden spezifischen Antikörpern garantiert.

Das Wesen der Erfindung setzt neben dem Einsatz von mit unterschiedlichen Markerenzymen markierten Immunreaktanten die Koimmobilisierung von Antikörpern als aktive Reaktionspartner und von antigenen Strukturen als passive Reaktionspartner an der festen Phase voraus. Die Immobilisierung von HIV-1/-2-Peptiden, die immundominante und hochgradig konservierte Strukturen aus den Transmembranproteinen beider Viren imitieren, realisiert einerseits aufgrund der geringen Molekülmasse der Peptide eine hohe Beladungsdichte (Molekülzahl pro Fläche) und damit eine hohe Empfindlichkeit für den Antikörpernachweis. Andererseits wird durch die geringe Molekülmasse der Peptide und die Reduzierung der Anzahl der unterschiedlichen Peptide auf jeweils ein einziges, die Adsorptionskapazität der festen Phase für den HIV-1/-2 Antikörpertest noch nicht erschöpft, und anti-HBs-Antikörper können zusätzlich adsorptiv gebunden werden.

In die beschichteten Kavitäten einer Mikrotiterplatte werden simultan Enzym-1 markierter Antikörper (z. B. Peroxidase-markierte monoklonale anti-HBs-Antikörper) und das zu untersuchende Serum dosiert. Während einer Inkubationsperiode von 30 bis 120 min (vorzugsweise 60 min) bei einer Reaktionstemperatur zwischen 20 und 40°C (vorzugsweise 37°C) können sowohl Antigen von den insolubilisierten Antikörpern gebunden werden, als sich andererseits auch Antikörper an die insolubilisierten Antigene binden. Bei Bindung von Antigen während dieser Inkubationsperiode binden sich gleichzeitig Enzym-1 markierte Antikörper an dieses Antigen. Nach bekannten Verfahren werden anschließend alle ungebundenen Proben- und Konjugatbestandteile aus den Kavitäten entfernt. Anschließend erfolgt die Substratreaktion zum Nachweis gebundener Peroxidaseaktivität unter Verwendung bekannter Chromogene und Substratpuffer (B. Porstmann, T. Porstmann und E. Nugel: J. Clin. Chem. Biochem. 19 (1981), 435-439), so daß nach Absorptionsmessung bereits an dieser Stelle entschieden werden kann, ob das gesuchte Antigen im Serum vorhanden ist oder nicht.

Nach Absaugen der Substratlösung und Spülung der Kavitäten mit Waschpuffer bekannter Zusammensetzung wird in die Kavitäten Enzym-2 markierter Antikörper (z. B. mit alkalischer Phosphatase markiertes anti-human IgG) dosiert. Nach einer Inkubationsperiode zwischen 30 und 120 min (vorzugsweise 60 min) bei einer Inkubationstemperatur zwischen 20 und 40°C (vorzugsweise 37°C) werden wiederum alle nicht gebundenen Reaktanten durch Spülen der Kavitäten mit Waschpuffer entfernt. Anschließend erfolgt der Nachweis der gebundenen Aktivität an alkalischer Phosphatase nach bekanntem Verfahren (E. Engvall, K. Jonsson und P. Perlmann: Biochem. et biophys. Acta 251 (1971), 427-434).

Bei Durchführung eines sukzessiven Zwei-Seiten Bindungsassays binden sich in der ersten Reaktionsphase genau wie beim simultanen Ein-Schritt Assay das gesuchte Antigen und/oder der gesuchte Antikörper. Im zweiten Reaktionsschritt werden nach Abtrennung der ungebundenen Reaktanten Enzym-1 markierter Antikörper und Enzym-2 markierter Antikörper als Gemisch dosiert und inkubiert. Die Substratreaktionen erfolgen dann sequentiell unmittelbar aufeinander.

Zum Nachweis des HBs-Antigens werden die definierten epitopdifferenten monoklonalen anti-HBsAg-Antikörper CB-HBsA und CB-HBsF verwendet. Sie werden aus den in der Hinterlegungsstelle des Zentralinstituts für Molekularbiologie (ZIM) der AdW der DDR, Berlin-Buch, registrierten Hybridomen H-CB-HBsA

3

EP 0 402 757 A2

(ZIM 0276) und H-CB-HBsF (ZIM 0279) gewonnen.

Das erfindungsgemäße Testbesteck zum simultanen Nachweis von verschiedenen Antigenen/Haptenen und/oder Antikörpern ist ein Festphasenimmunoassay und besteht aus:

- der festen Phase, zum Beispiel Mikrotiterplatten, Röhrchen, Kugeln, Zylinder, Scheiben u. ä., mit koimmobilisierten Reaktionspartnern, zum Beispiel verschiedenartigen Bindern (Antikörpern unterschiedlicher Spezifität) zum Nachweis diffe renter Antigene oder verschiedenartigen Liganden (unterschiedliche Antigene oder Haptene) zum Nachweis differenter Antikörper oder Bindern und Liganden (Antikörpern und Antigenen beziehungsweise Haptene) zum Nachweis von Antigenen/Haptenen und spezifischen Antikörpern.

- Peroxidase-markierten Bindern (Antikörpern) oder Liganden (Antigenen/Haptenen) zum Nachweis von Antigenen oder Antikörpern nach einem nichtkompetitiven oder kompetitiven Verfahren.

- Alkalische Phosphatase-markierten Bindern (Antikörpern) oder Liganden (Antigenen/Haptenen) zum Nachweis von Antigenen/Haptenen nach einem nicht-kompetitiven oder kompetitiven Verfahren, beziehungsweise zum Nachweis von Antikörpern nach einem nicht-kompetitiven oder kompetitiven Verfahren.

- Substratgemisch zum Nachweis von Peroxidase.

- Substratgemisch zum Nachweis von alkalischer Phosphatase

- Stoppreagens für die Peroxidase-Reaktion

- Stoppreagens für die alkalische Phosphatase-Reaktion

- Verdünnungsmedium für Proben und Konjugate (Konzentrat)

- Waschpuffer (Konzentrat)

- Kontrollmaterialien

Ausführungsbeispiel

Methodik

Zwei epitopdifferente monoklonale anti-HBs-Antikörper, CB-HBsA und CB-HBs F (gereinigte Syntheseprodukte der Hybridomzellinien ZIM 0276 und 0279), werden in Konzentrationen von 1 mg/l und 7 mg/l zusammen mit dem HIV-1-spezifischen Peptid und dem HIV-2-spezifischen Peptid, beide eingestellt auf eine Konzentration von 5 mg/l in 0,1 mol/l Karbonatpuffer, pH 9,5, nach bekannten Verfahren (K. Catt und G.W. Traeger, Science 158 (167), 1570-1572) über Nacht bei 20° C-25° C an die Oberfläche von Polystyrol-Mikrotiterplatten (Scopyron Mikrotestplatte, VEB Polyplast Halberstadt, DDR) adsorbiert. Nach Absaugen und einmaliger Spülung der Kavitäten mit dest. Wasser erfolgt eine Nachbenetzung mit 10 % (v/v) Schafserum und 5 1 (w/v) Saccharose, gelöst in 0.01 mol/l Phosphatpuffer, pH 7,2 über 60 min bei 20 - 25° C. Anschließend wird die Flüssigkeit in den Kavitäten abgesaugt, und die Platten werden 30 min bei 37° C mit Luftumwälzung im Brutschrank nachgetrocknet. Vor Testbeginn werden die Kavitäten einer beschichteten Platte einmal mit Waschpuffer (0,01 mol/l Phosphatpuffer, pH 7,2, 0,3 mol/l NaCl, 0,1 % (v/v) Tween 20) gespült und anschließend trocken gesaugt.

In alle Kavitäten werden 50 $\mu$l Peroxidase-markierter monoklonaler anti-HBs Antikörper (CB-HBsA), eingestellt mit Verdünnungsmedium (Waschpuffer, 10 % (v/v) Schafserum, 0,5 % (v/v) Mausserum) auf eine Konzentration von 2 mg/l, dosiert. Anschließend werden jeweils 50 $\mu$l des zu untersuchenden Serums in die Kavitäten gegeben mit Ausnahme der Positionen A1 bis A6. In die Position A1 werden 50 $\mu$l Verdünnungsmedium, in die Positionen A2,3,4 jeweils 50 $\mu$l negatives Kontrollserum (NKS) und in die Positionen A5,6 jeweils 50 $\mu$l positives Kontrollserum (HBsAg-haltig und anti-HIV-antikörperhaltig) dosiert. Nach Abdeckung der Platte erfolgt die Inkubation über 60 min bei 37° C. Nach Abtrennung der ungebundenen Reaktanten durch fünfmaliges Spülen mit Waschpuffer erfolgt die Substratreaktion mit o-Phenylendiamin und $H_2O_2$ nach bekannten Verfahren (T. Porstmann, B. Porstmann, R. Wietschke und E. Egger: J. Clin. Chem. Clin. Biochem. 23 (1985), 41-45) durch Zugabe von 200 $\mu$l pro Kavität. Nach einer Reaktionszeit von 30 min erfolgt ohne Reaktionsabbruch die bichromatische Extinktionsmessung bei 450 und 690 nm. Nach automatischem Vergleich der durch die untersuchten Seren hervorgerufenen Extinktionen mit der für HBsAg ermittelten Grenzwertextinktion von 0,03 Extinktionseinheiten werden bei eindeutig negativem oder positivem Befund die Kavitäten leergesaugt und einmal mit Waschpuffer gewaschen. Bei Extinktionen im "Grauzonenbereich" werden 150 $\mu$l Substratlösung aus den entsprechenden Kavitäten der ELISA-Platte zusammen mit den Substratlösungen aus den Kavitäten A1 bis A6 entnommen und in die Kavitäten einer raten unbehandelten Mikrotiterplatte dosiert. Dazu werden 100 $\mu$l 2,0 mol/l $H_2SO_4$ pro Kavität dosiert, die Kavitäten anschließend bichromatisch bei 492 und 690 nm vermessen und mit der Grenzwertextinktion für HBsAg nach Reaktionsstopp (0,06 Extinktionseinheiten) verglichen.

4

EP 0 402 757 A2

Für den zweiten Teil werden 100 $\mu$l mit alkalischer Phosphatase markiertes anti-human IgG, eingestellt mit Verdünnungsmedium auf eine Konzentration von 0,3 mg/l IgG, in alle Kavitäten der ELISA-Platte dosiert und 60 min bei 37° C inkubiert. Nach Entfernung der ungebundenen Reaktanten durch fünfmaliges Spülen der Kavitäten mit Waschpuffer werden 100 $\mu$l Substrat mit p-Nitrophenylphosphat in Diethanolaminpuffer nach bekannten Verfahren (E. Engvall, K. Jonsson und P. Perlmann: Biochem. et biophy. Acta 251 (1971), 427-434) pro Kavität dosiert. Der Reaktionsabbruch erfolgt nach 30 min Inkubation bei 20° C durch Zugabe von 100 $\mu$l 0,5 mol/l $Na_2CO_3$ pro Kavität. Die Extinktionsmessung wird bichromatisch bei 405 und 690 nm gegen Kavität A1 als Leerwert durchgeführt. Die durch die untersuchten Seren hervorgerufenen Extinktionen werden automatisch mit der für antiHIV1/2 ermittelten Grenzwertextinktion (x Ext. NKS 0.300) verglichen. Die einzelnen Arbeitsschritte mit den entsprechenden Reaktanten sind in Tabelle 1 aufgelistet.

Ermittlung der Nachweisgrenze für HBsAg und der Nachweisempfindlichkeit für anti-HIV-Antikörper im Verfahren zur simultanen differenzierten Erregerbestimmung unter Einfluß unterschiedlicher zur Koimmobilisierung eingesetzten Peptidkonzentrationen. 100 $\mu$l des Gemisches der monoklonalen anti-HBs-Antikörper (CB-HBsA und CB-HBsF) werden ohne und mit HIV-1/-2-Peptiden in den Konzentrationen 1,2,3,4,5 und 10 mg/l zur Beschichtung der Kavitäten eingesetzt.

Versuch 1

In diesen Kavitäten werden die Extinktionen für verschiedene Konzentrationen des HBsAg-Referenzserums ermittelt und mit dem Extinktionsmittelwert plus seiner dreifachen Standardabweichung des Verdünnungsmediums verglichen.

Versuch 2

In diesen Kavitäten werden die Extinktionen für verschiedene Verdünnungen des anti-HIV-1-antikörperhaltigen gepoolten Serums mit dem Extinktionsmittelwert plus seiner dreifachen Standardabweichung des Verdünnungsmediums verglichen.

Versuch 3

In diesen Kavitäten werden die Extinktionen für die geometrische Verdünnung eines Gemisches aus dem gepoolten anti-HIV-1-antikörperhaltigen Serums und dem HBsAg-Standard (positives Kontrollserum, PKS) mit den Extinktionen verglichen, die durch Titration des HBsAg-Referenzserums allein (Versuch 1) und des gepoolten anti-HIV antikörperhaltigen Serums allein (Versuch 2) erzielt wurden.

Versuch 4

Unter Festlegung der Grenzwertextinktion für HBsAg ohne Reaktionsstopp bei 450/690 nm = 0,030 (entspricht einer unteren Nachweisgrenze von 1,25 $\mu$g HBsAg/l) und einer Grenzwertextinktion für anti-HIV Antikörper = 0,300 plus dem Extinktionsmittelwert des negativen Kontrollserums (NKS) bei 405/690 nm, wurden Spezifität und Sensitivität des Kombitestes an 1000 Blutspendern, 710 HIV-1 und 3 HIV-2 Infizierten (Stadium 1 - 4, CDC-Klassifikation) im Vergleich mit einem anti-HIV ELISA der ersten Generation getestet.

Ausführungsbeispiel 2

Methodik

In alle Kavitäten mit den koimmobilisierten monoklonalen anti-HBs-Antikörpern und HIV-1/HIV-2-Peptiden (siehe Ausführungsbeispiel 1) werden 50 $\mu$l des zu untersuchenden Serums und 50 $\mu$l Verdünnungsmedium (siehe Ausführungsbeispiel 1) als Gemisch dosiert mit Ausnahme der Positionen A1 bis A6. In die Position A1 werden 100 $\mu$l Verdünnungsmedium, in die Positionen A2, A3, A4 werden 50 $\mu$l negatives Kontrollserum (NKS), in die Positionen A5 und A6 jeweils 50 $\mu$l positiv reagierendes Kontrollserum (PKS)

5

zusammen mit 50 $\mu$l Verdünnungsmedium als Gemisch dosiert. Nach Abdeckung der Platte erfolgt die Inkubation über 60 min bei 37° C. Anschließend werden die ungebundenen Reaktanten durch fünfmaliges Spülen der Kavitäten mit Waschpuffer entfernt. Daraufhin erfolgt die Dosierung von 100 $\mu$l eines Konjugatgemisches pro Kavität. Das Konjugatgemisch besteht aus Peroxidase-markierten monoklonalen anti-HB-Antikörpern (CB-HBsA), eingestellt auf eine Konzentration von 2 mg/l und anti-human IgG, markiert mit alkalischer Phosphatase, eingestellt auf eine Konzentration von 0,3 mg/l IgG. Das Konjugatgemisch wird 60 min bei 37° C inkubiert. Ungebundene Konjugatbestandteile werden durch fünfmaliges Spülen der Kavitäten mit Waschpuffer entfernt. Anschließend wird der Aktivitätsnachweis für gebundene alkalische Phosphatase durch Dosierung von 100 $\mu$l Substrat mit p-Nitrophenylphosphat in Diethanolaminpuffer nach bekannten Verfahren (Literatur, siehe Ausführungsbeispiel 1) durchgeführt.

Die Enzymreaktion wird nach 30 min durch kompetitive Substrathemmung, indem 100 $\mu$l 0,1 mol/l $Na_2HPO_4$ mit 0,25 mol/l Phenylphosphat pro Kavität dosiert werden, abgebrochen.

Unmittelbar nach Reaktionsabbruch erfolgt die bichromatische Extinktionsmessung bei 405 und 690 nm. Die durch die Seren hervorgerufenen Extinktionen werden automatisch mit der für anti-HIV1/-2 ermittelten Grenzwertextinktion (x Ext. NKS + 0,300) verglichen. Anschließend werden die Kavitäten leergesaugt und einmal mit Waschpuffer gewaschen. Jetzt erfolgt der Nachweis von HBsAg über die Bestimmung der Peroxidase-aktivität in den Kavitäten durch Dosierung von 100 $\mu$l Substratlösung mit o-Phenylendiamin und $H_2O_2$ pro Kavität. Nach 30 min Reaktionszeit wird die Reaktion durch Dosierung von 100 $\mu$l 1 mol/l Schwefelsäure, 0,05 mol/l Natriumsulfit abgebrochen und das gebildete farbgebende Produkt stabilisiert (Literatur, siehe Ausführungsbeispiel 1). Im Anschluß daran erfolgt die bichromatische Extinktionsmessung bei 492 und 690 nm. Die durch die untersuchten Seren hervorgerufenen Extinktionen werden automatisch mit der für HBsAg ermittelten Grenzwertextinktion (x Ext. NKS 0,03) verglichen. Die einzelnen Arbeitsschritte mit den entsprechenden Reaktanten sind in Tabelle 2 aufgelistet.

Versuch

In den Kavitäten mit koimmobilisierten anti-HBs-Antikörpern und HIV-1/-2-Peptiden werden die Extinktionen eines Gemischs aus dem gepoolten anti-HIV-1-antikörperhaltigem Serum und dem HBsAg-Standard (positives Kontrollserum, PKS) mit den Extinktionen verglichen, die durch Titration des HBsAg-Referenzserums allein und des gepoolten anti-HIV-1-antikörperhaltigen Serums allein erzielt wurden.

Ergebnis

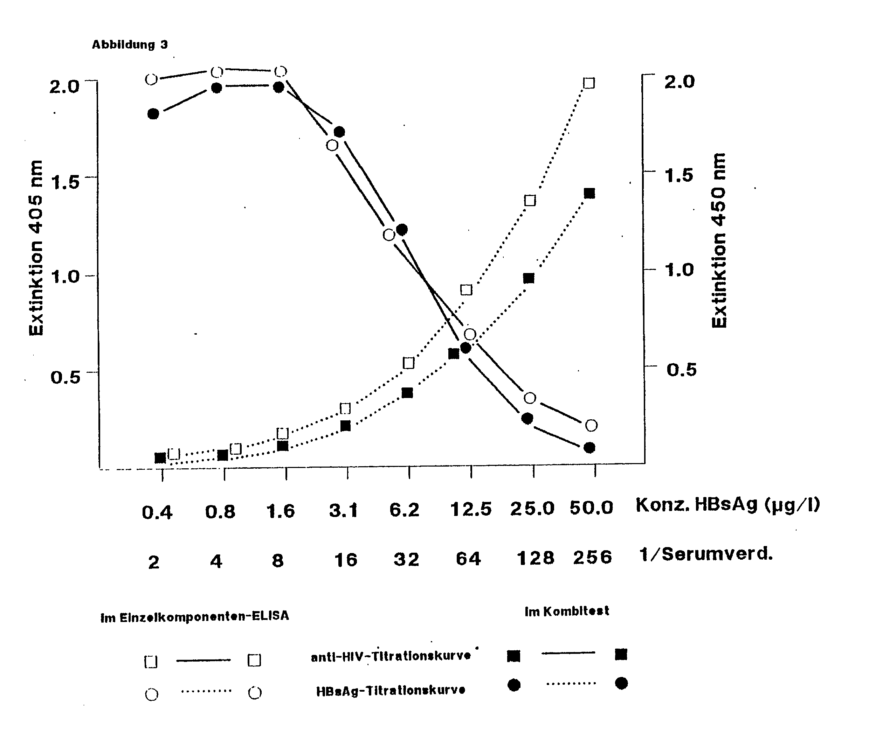

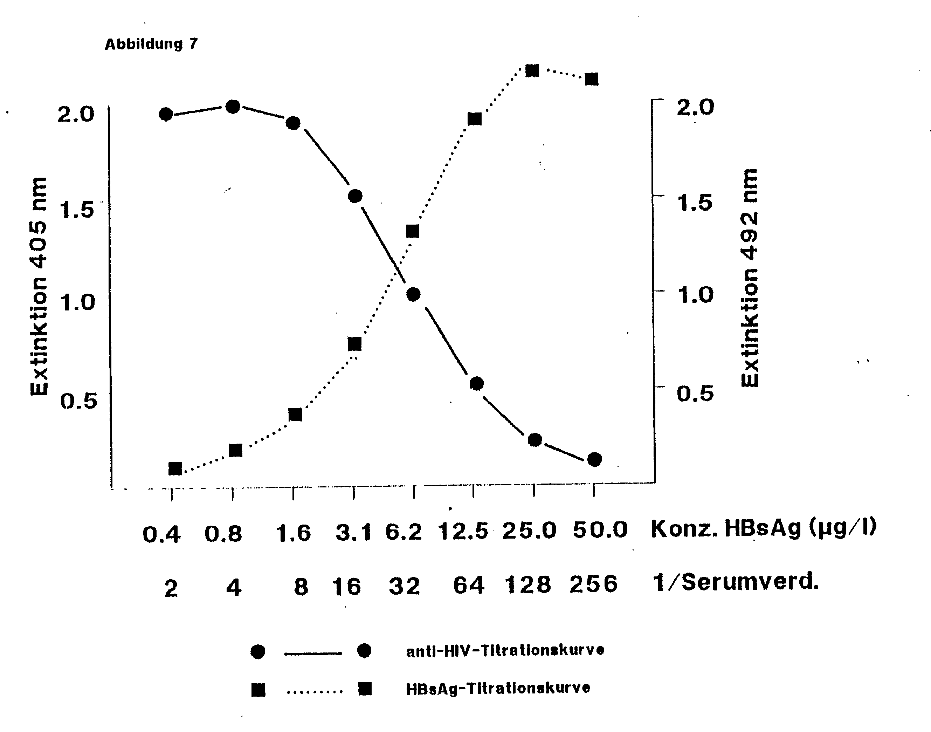

Anti-HIV-Antikörper werden in Abwesenheit von HBsAg genauso empfindlich nachgewiesen wie bei Anwesenheit von HBsAg. Das gleiche trifft auch für die Nachweisempfindlichkeit von HBsAg in An- und Abwesenheit von anti-HIV-1-Antikörpern zu. Während die Nachweisempfindlichkeit für anti-HIV-1-Antikörper in der Testvariante nach Ausführungsbeispiel 2 gegenüber der Testvariante nach Ausführungsbeispiel 1 unverändert bleibt (vergleiche Abbildung 3), steigt die Nachweisempfindlichkeit für HBsAg bei Ausführungsbeispiel 2 gegenüber Ausführungsbeispiel um 2 Titerstufen. Mit der Testvariante, beschrieben in Ausführungsbeispiel 2, beträgt die untere Nachweisgrenze für HBsAg weniger als 0,5 $\mu$g/l (Abb. 7).

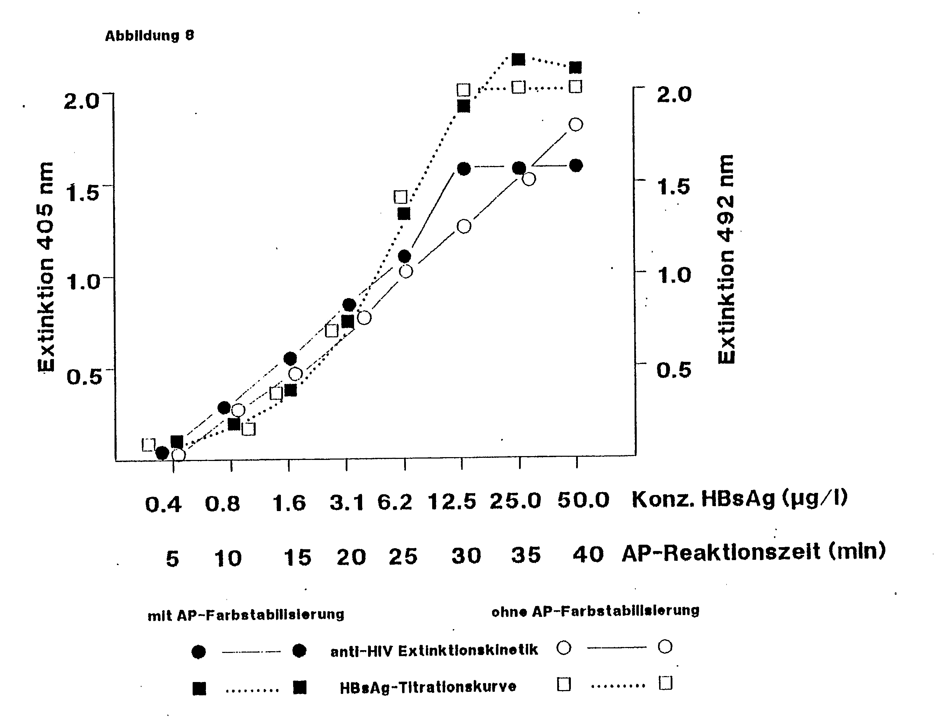

Die kompetitive Substrathemmung durch Zusatz von Nitrophenylphosphat und/oder Dinatriumhydrogenphosphat limitiert den weiteren Umsatz von para-Nitrophenylphosphat komplett un stabilisiert somit die erreichte Farbintensität ohne den anschließenden Aktivitätsnachweis für die Peroxidase zu beeinflussen (Abb. 8).

Ergebnisse

Versuch 1

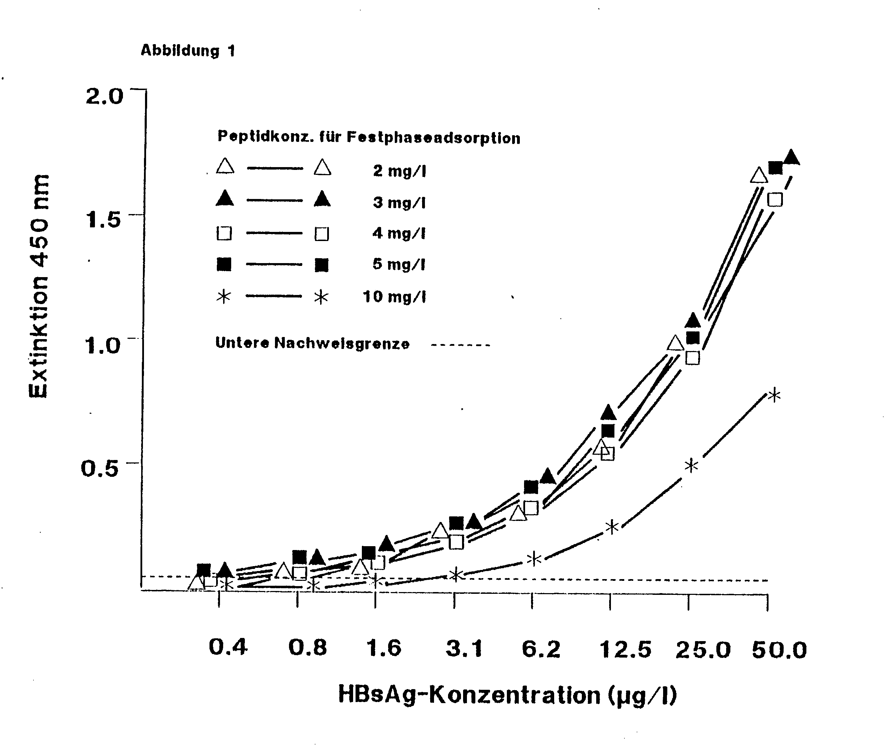

Bei Koimmobilisierung von HIV-Peptiden und anti-HBs-Antikörpern bleibt die Empfindlichkeit des HBsAg-Nachweises bis zu einer HIV-1- und HIV-2-Peptidkonzentration von jeweils 5 mg/l unbeeinflußt. Lediglich bei Einsatz von Peptidkonzentrationen von 10 mg/l zur Festphaseadsorption verringert sich die Nachweisempfindlichkeit für HBsAg über die koimmobilisierten Antikörper um den Faktor 3 (Abb. 1).

Die Spezifität des HBsAg-Testes wird durch die Koimmobilisierung der Peptide in den verschiedenen Konzentrationen nicht beeinflußt (s.u.). Bei Titration der Referenzseren für HBsAg wurden die beiden Subtypen ad und ay nahezu gleich empfindlich erfaßt.

Versuch 2

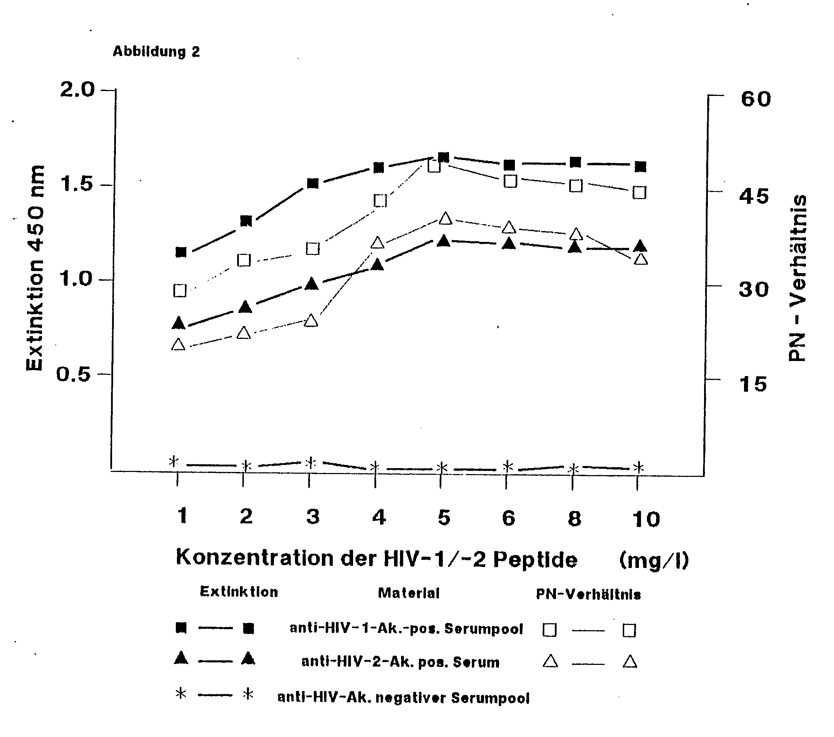

Bei Koimmobilisation von anti-HBs-Antikörpern und HIV-1 und HIV-2-Peptiden erweist sich lediglich der anti-HIV-Antikörpernachweis von der Peptidkonzentration abhängig, während der HBsAg-Nachweis unbeeinflußt bleibt. Mit steigenden Peptidkonzentrationen (1 bis 5 mg/l), die zur Insolubilisierung eingesetzt werden, steigt die Nachweisempfindlichkeit für anti-HIV-Antikörper um mindestens eine Titerstufe. Bei dem Einsatz von 10 mg/l des jeweiligen Peptides zur Festphasenbenetzung ist jedoch gegenüber der Konzentration von 5 mg/l keine weitere Steigerung der Nachweisempfindlichkeit mehr erzielbar, gemessen an den Extinktionsquotienten (PN-Verhältnis) der anti-HIV Antikörper-positiven und -negativen Seren (Abb. 2).

Versuch 3

Das sowohl HBsAg- als auch anti-HIV-antikörperhaltige Serum (positives Kontrollmaterial, PKS) reagiert im ersten und im zweiten Teil des Kombitestes positiv. Ein Serumpool, der weder HBsAg noch anti-HIV-Antikörper enthält (negatives Kontrollmaterial, NKS), überschreitet in beiden Teilen des Kombitestes nicht die festgelegten Grenzwertextinktionen. Das PKS wird hinsichtlich seines HBsAg-Gehaltes und seines Gehaltes an anti-HIV-Antikörpern im Kombitest genauso empfindlich bestimmt wie im simultanen Ein-Schritt-EIA zum HBsAg-Nachweis oder anti-HIV-1-ELISA auf Peptidbasis ohne Koimmobilisierung der jeweils anderen Reaktionskomponente (Abb. 3).

Versuch 4

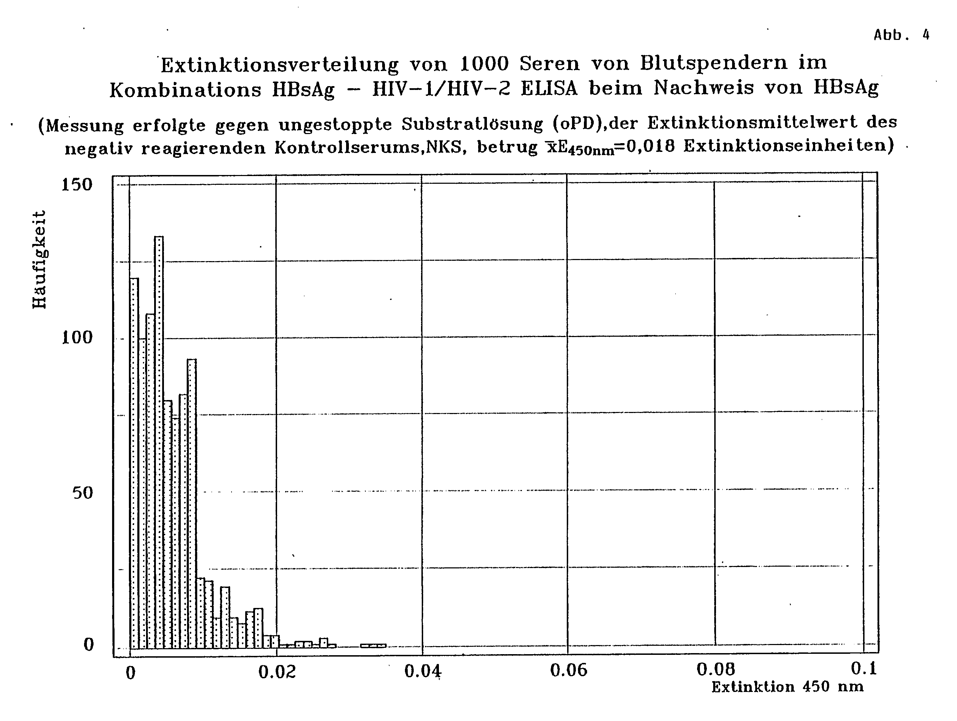

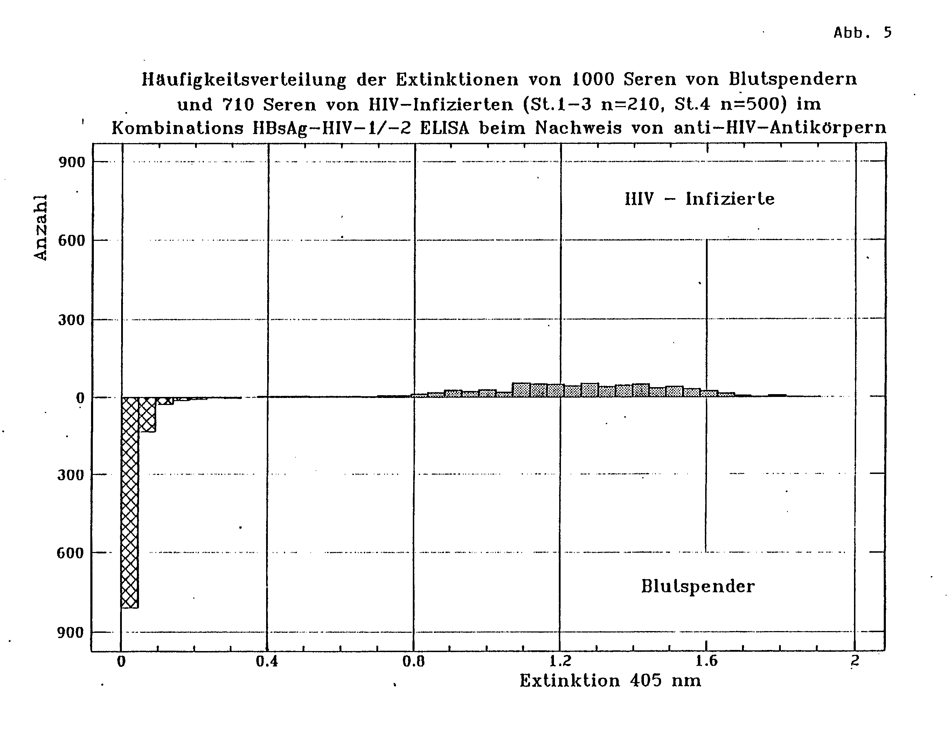

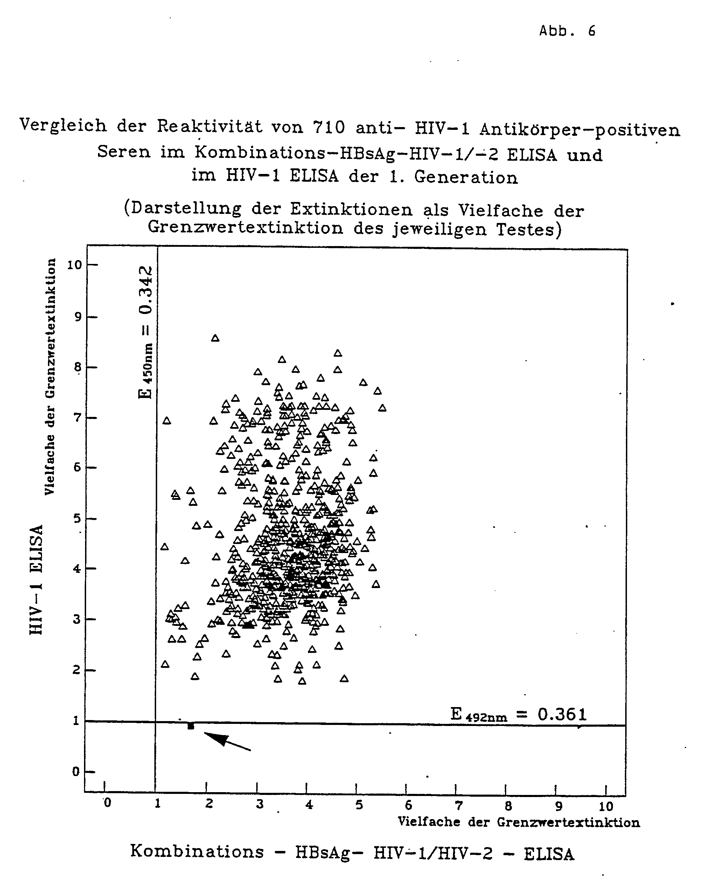

Unter Verwendung der festgelegten Extinktionsgrenzwerte für die HBsAg-Bestimmung und den Nachweis von anti-HIV-Antikörpern wies der Kombitest folgende Spezifitäten auf:
Spezifität HBsAg-Bestimmung = 100 %
Spezifität anti-HIV-Antikörperbestimmung = 100 %
Die Extinktionsverteilungen sind in den Abbildungen 4 und 5 dargestellt.
Die Sensitivität des Kombitestes bezüglich der Erfassung von anti-HIV-1-Antikörpern beträgt 99,86 %. Das einzige als anti-HIV-antikörpernegativ bestimmte Serum von einem AIDS-Patienten im Finalstadium wurde auch in drei weiteren kommerziellen anti-HIV Testen falsch negativ bestimmt. Im Westernblot (Du Pont) ergab sich bei einer 1 : 100 Verdünnung des Serums eine schwache Antikörperreaktivität gegen gp160, gp120 und p24. Dagegen wurde eine Serumprobe aus einer frühen HIV-Infektionsphase bei Vergleichsuntersuchungen mit dem Kombitest im Gegensatz zu einem Test der ersten Generation als richtig positiv bestimmt (Abb. 6).

EP 0 402 757 A2

Tabelle 1

| Ablauf der Arbeitsschritte bei Ausführungsbeispiel 1 | | | |
|---|---|---|---|
| | Arbeitsschritt | Reaktante | Zeitdauer |
| 1. | Waschen (1x) | Waschpuffer | 1 min |
| 2. | Dosierung | Serum/POD-Konjugat-Gemisch | 10 min |
| 3. | Inkubation | | 60 min |
| 4. | Waschen (5x) | Waschpuffer | 5 min |
| 5. | Dosierung | POD-Substratlösung | 2 min |
| 6. | Inkubation | | 30 min |
| 7. | Messung bei 450 nm | | 1 min |
| 8. | Waschen (1x) | | 1 min |
| 9. | Dosierung | AP-Konjugat | 2 min |
| 10. | Inkubation | | 60 min |
| 11. | Waschen (5x) | Waschpuffer | 5 min |
| 12. | Dosierung | AP-Substratlösung | 2 min |
| 13. | Inkubation | | 30 min |
| 14. | Dosierung | Natronlauge | 3 min |
| 15. | Messung bei 405 nm | | 1 min |

Tabelle 2

| Ablauf der Arbeitsschritte bei Ausführungsbeispiel 2 | | | |
|---|---|---|---|
| | Arbeitsschritt | Reaktante | Zeitdauer |
| 1. | Waschen (1x) | Waschpuffer | 1 min |
| 2. | Dosierung | Serum/Verdünnungsmedium-Gemisch | 10 min |
| 3. | Inkubation | | 60 min |
| 4. | Waschen (5x) | Waschpuffer | 5 min |
| 5. | Dosierung | AP-POD-Konjugatgemisch | 2 min |
| 6. | Inkubation | | 60 min |
| 7. | Waschen (5x) | | 5 min |
| 8. | Dosierung | AP-Substratlösung | 2 min |
| 9. | Inkubation | | 30 min |
| 10. | Dosierung | Stopplösung | 2 min |
| 11. | Messung bei bei 405 nm | | 1 min |
| 12. | Waschen (1x) | Waschpuffer | 1 min |
| 13. | Dosierung | POD-Substratlösung | 2 min |
| 14. | Inkubation | | 30 min |
| 15. | Dosierung | Schwefelsäure | 2 min |
| 16. | Messung bei 492 nm | | 1 min |

**Ansprüche**

1. Verfahren zur simultanen differenzierten Bestimmung unterschiedlicher Antigene/Haptene und/oder Antikörper in einem Testansatz, in dem aufgrund unterschiedlicher Nachweisreaktionen die An- oder Abwesenheit der gesuchten Antigene und/oder Antikörper bestimmt werden kann, dadurch gekennzeichnet, daß in einem Festphasenimmunoassay die jeweils zu bestimmenden Substanzen spezifischen und mit unterschiedlichen Enzymen markierten Antikörper oder spezifischen Antigene, simultan oder sequentiell inkubiert und gegebenenfalls die Substratreaktionen sequentiell durchgeführt werden.

8

2. Verfahren nach Anspruch 1 dadurch gekennzeichnet, daß als Festphasenimmunoassay eine beliebige Kombination folgenden Assays durchgeführt wird:
- Zweiseitenassays
- Antiglobulin- oder Sandwichassays
- Kompetitionsassays
- Captureassays
- Brückenantikörperassays

3. Verfahren nach Anspruch 1 oder 2, dadurch gekennzeichnet, daß die für den Nachweis der zu bestimmenden Antigen und/oder Antikörper spezifischen Antikörper und/oder Antigene mit unterschiedlichen Enzymen markiert sind.

4. Verfahren nach Anspruch 3, dadurch gekennzeichnet, daß die Enzyme Peroxidase oder alkalische Phosphatase sind.

5. Verfahren nach einem der Ansprüche 1 bis 3, dadurch gekennzeichnet, daß als Markersubstanzen Enzymkombinationen gewählt werden, die während der ersten Substratreaktion weder die gebildeten Immunkomplexe denaturieren noch das gebundene zweite Markerenzym in seiner Aktivität mindern.

6. Verfahren nach einem der Ansprüche 1 bis 5, dadurch gekennzeichnet, daß zum Nachweis von HBsAg und Antikörpern gegen HIV-1 und HIV-2 für den HBsAg-Nachweis monoklonale Antikörper mit Spezifität für die s- und pre-s Region und für den anti-HIV-Antikörpernachweis nur jeweils ein Peptid aus dem Transmembranbereich des jeweiligen Virusstammes eingesetzt wird.

7. Verfahren nach Anspruch 1 bis 5, dadurch gekennzeichnet, daß bei Aufeinanderfolge des Aktivitätsnachweises von alkalischer Phosphatase und Peroxidase, das gebildete farbgebende Produkt, bei der der Peroxidase vorangehenden Reaktion der alkalischen Phosphatase durch kompetitive Substrathemmung stabilisiert wird, wodurch weder die Immunkomplexe, über die die Peroxidase gebunden ist, gespalten werden noch die Peroxidase als Markerenzym in ihrer Aktivität beeinflußt wird.

8. Testbesteck zur Durchführung des Verfahrens nach einem der Ansprüche 1 bis 7 bestehend aus a) einer festen Phase mit koimmobilisierten HIV-1- und -2-Peptiden und monoklonalen anti-HBs-Antikörpern, b) mit Peroxidase markierten monoklonalen anti-HBs-Antikörpern, c) mit alkalischer Phosphatase markierten anti-human IgG-Antikörpern, d) einem Substratgemisch zum Nachweis von Peroxidase, e) einem Substratgemisch zum Nachweis von alkalischer Phosphatase, f) Stoppreagens für die Peroxidase-Reaktion, g) Stoppreagens für die alkalische Phosphatase-Reaktion, h) Verdünnungsmedium für Proben und Konjugate (als Konzentrat), i) Waschpuffer (als Konzentrat).

Abbildung 1

Peptidkonz. für Festphaseadsorption

△  2 mg/l
▲  3 mg/l
□  4 mg/l
■  5 mg/l
✳  10 mg/l

Untere Nachweisgrenze - - - - - -

Extinktion 450 nm

2.0
1.5
1.0
0.5

0.4  0.8  1.6  3.1  6.2  12.5  25.0  50.0

HBsAg-Konzentration (µg/l)

Abbildung 2

Extinktion 450 nm

PN – Verhältnis

Konzentration der HIV-1/-2 Peptide    (mg/l)

EP 0 402 757 A2

| Extinktion | Material | PN-Verhältnis |
|---|---|---|
| ■ —— ■ | anti-HIV-1-Ak.-pos. Serumpool | □ —— □ |
| ▲ —— ▲ | anti-HIV-2-Ak. pos. Serum | △ —— △ |
| * —— * | anti-HIV-Ak. negativer Serumpool | |

Abbildung 3

Extinktion 405 nm

Extinktion 450 nm

Konz. HBsAg (µg/l)

0.4   0.8   1.6   3.1   6.2   12.5   25.0   50.0

2     4     8     16    32    64     128    256    1/Serumverd.

Im Einzelkomponenten-ELISA                    Im Kombitest

anti-HIV-Titrationskurve

HBsAg-Titrationskurve

EP 0 402 757 A2

Abb. 4

## Extinktionsverteilung von 1000 Seren von Blutspendern im Kombinations HBsAg – HIV–1/HIV–2 ELISA beim Nachweis von HBsAg

(Messung erfolgte gegen ungestoppte Substratlösung (oPD),der Extinktionsmittelwert des negativ reagierenden Kontrollserums,NKS, betrug $\overline{x}E_{450nm}=0,018$ Extinktionseinheiten)

Häufigkeit

Extinktion 450 nm

EP 0 402 757 A2

Abb. 5

Häufigkeitsverteilung der Extinktionen von 1000 Seren von Blutspendern
und 710 Seren von HIV-Infizierten (St.1-3 n=210, St.4 n=500) im
Kombinations HBsAg-HIV-1/-2 ELISA beim Nachweis von anti-HIV-Antikörpern

EP 0 402 757 A2

Abb. 6

Vergleich der Reaktivität von 710 anti- HIV-1 Antikörper-positiven
Seren im Kombinations-HBsAg-HIV-1/-2 ELISA und
im HIV-1 ELISA der 1. Generation

(Darstellung der Extinktionen als Vielfache der
Grenzwertextinktion des jeweiligen Testes)

Kombinations − HBsAg− HIV-1/HIV-2 − ELISA

Abbildung 7

anti-HIV-Titrationskurve

HBsAg-Titrationskurve

EP 0 402 757 A2

Abbildung 8